# FASCICULE DE BREVET EUROPEEN

(11) **EP 2 491 893 B1**
(45) Date de publication et mention de la délivrance du brevet: **07.01.2015**
(21) Numéro de dépôt: 12156577.4
(22) Date de dépôt: 22.02.2012
(51) Int. Cl.: A61F 2/30, A61B 17/84, A61B 17/86, A61B 17/17, A61F 2/32, A61F 2/36

(54) **Implant de reprise fémorale et ancillaire de mise en oeuvre chirurgicale**
Implantat zur Wiederherstellung von Oberschenkelknochen, und chirurgisches Instrument zum Einsetzen des Implantats
Femoral repair implant and ancillary for surgical implementation

(30) Priorité: 23.02.2011 FR 1151479
(43) Date de publication de la demande: 29.08.2012
(73) Titulaire: BIOMET, 26000 Valence (FR)
(72) Inventeur: Augoyard, Marc, 69160 Tassin-La-Demi-Lune (FR); Melere, Gilles, 74370 Saint-Martin-De-Bellevue (FR); Millon, Joseph, 73490 La Ravoire (FR); Passot, Jean-Paul, 42100 Saint-Etienne (FR); Peyrot, Jacques, 69160 Tassin-La-Demie-Lune (FR); Relave, Marc, 42160 Andrezieux Boutheon (FR); Ameil, Marc, 51100 Reims (FR); Braud, Gérard, 01000 Bourg-en-Bresse (FR); Catonne, Yves, 75011 Paris (FR); Chauveaux, Dominique, 33200 Bordeaux (FR); Jouanin, Thierry, 26200 Montelimar (FR); Basso, Maurice, 83400 Giens-Hyeres (FR); Simottel, Jean-Claude, 76850 Cottevrard (FR); Clavel, Sébastien, 26120 Châteaudouble (FR); Giffard, Lénaïc, 69230 Saint-Genis-Laval (FR); Charret, Philippe, 69270 Fontaines-sur-Saone (FR); Debiesse, Jean-Louis, 38200 Vienne (FR); Dupre Latour, Laurent, 42580 L'Etrat (FR); Eyraud, Guy, 38150 Ville-sous-Anjou (FR); Fayard, Jean-Philippe, 42170 Saint Just-Saint Rambert (FR); Hulin, Paul-Henri, 89000 Auxerre (FR); Lecuire, François, 83400 Giens-Hyeres (FR)
(74) Mandataire: Thibault, Jean-Marc

(56) Documents cités:
- EP-A2- 0 159 510
- DE-U1- 29 907 259
- FR-A1- 2 674 744
- FR-A1- 2 855 395
- US-A- 4 714 470

## Description

La présente invention concerne le domaine des implants orthopédiques et, plus particulièrement, des prothèses mises en oeuvre pour réhabiliter des articulations endommagées du corps humain.

Les caractéristiques du préambule de la revendication 1 sont connues du document EP-A-0159510.

L'objet de l'invention concerne notamment les prothèses de hanche et notamment les implants fémoraux qui sont mis en place après résection du col du fémur, dans les interventions destinées à réhabiliter l'articulation coxo-fémorale.

Dans le domaine technique ci-dessus, on connaît un très grand nombre de prothèses de hanche et implants destinés à assurer une réhabilitation de l'articulation de la hanche dans les meilleures conditions et pour des durées les plus longues possibles.

De façon générale, les prothèses de hanche peuvent être classées en deux catégories selon qu'elles sont implantées avec ou sans présence d'un liant intermédiaire, tel qu'un ciment.

Il est malheureusement constant que, consécutivement à l'implantation de telles prothèses il est, dans certains cas, nécessaire de procéder à une révision de l'implantation réalisée qui connaît des désordres exigeant l'extraction et le remplacement de la prothèse. Les causes de tels désordres sont nombreuses et peuvent provenir d'une implantation première non optimale, d'une dégradation du liant lorsque celui-ci est présent, de l'installation d'une infection, d'un descellement ou d'une dégradation naturelle du matériel osseux abritant la prothèse.

Il est alors nécessaire de procéder à la mise en place d'une nouvelle prothèse, dite de reprise, dans le fémur du patient, fémur dont l'état s'est dégradé du fait des désordres précédents. Ces dégradations peuvent notamment relever de l'état du matériel osseux, voire des déformations ou des fractures du fémur ou, encore, de la dégradation de son extrémité supérieure d'appui résultant de la résection du col du fémur intervenue lors de la première implantation.

Dans les cas de révisions de prothèses de hanches, une remise en état du canal médullaire du fémur dont la partie interne a pu être dégradée est nécessaire.

Il en résulte malheureusement dans la majorité des cas un relatif agrandissement du canal médullaire, qui augmente les problèmes de stabilité d'implantation de l'implant de reprise, en particulier lors de toutes les étapes préalables de réduction de la hanche pour vérifier les données chirurgicales. Ainsi, du fait principalement de ces problèmes de stabilité des implants de reprise, les opérations de reprise de prothèses de hanches sont souvent difficiles pour les praticiens. Ces difficultés sont d'autant plus grandes que dans certains cas, l'absence de repères osseux pose des problèmes de positionnement de la tige notamment pour ce qui est de sa position angulaire et de son enfoncement.

L'objet de l'invention est de proposer une prothèse de reprise qui permettent d'améliorer la stabilité de la prothèse lors des opérations de réduction, ainsi que de faciliter l'implantation définitive de la prothèse de reprise et le rétablissement de l'intégrité de l'articulation coxo-fémorale.

Pour atteindre les objectifs ci-dessus, l'invention propose un implant fémoral de reprise, comprenant une tige liée par un col à une tête, ladite tige comportant, à partir du col, une partie métaphysaire, prolongée par une queue. La tige possède un axe principal (A-A') faisant un angle (a) avec un axe secondaire (B-B') du col. Par ailleurs, selon une caractéristique essentielle de l'invention, la queue présente deux faces planes parallèles entre elles et au plan de référence sagittal anatomique et reliés l'une à l'autre par deux faces non planes munies d'éléments longitudinaux anti-rotation.

Selon une variante préférée de réalisation de l'implant de l'invention, les éléments longitudinaux anti-rotation sont constitués d'ailettes ou de bourrelets saillants à la surface des faces non-planes de la queue de la tige.

Selon une forme de réalisation, l'implant fémoral de' l'invention comporte au moins deux éléments longitudinaux anti-rotation, et de préférence entre deux et six éléments longitudinaux anti-rotation.

Toujours selon l'invention, les éléments anti-rotation s'étendent depuis la base de la queue jusqu'à sensiblement l'extrémité distale de la queue.

Selon une forme de réalisation, la partie métaphysaire comporte :
- deux faces planes,
- un bord interne concave, raccordant le col à la queue,
- et un bord supéro-externe prenant naissance à partir d'une surface supérieure plane de raccordement au col.

Par ailleurs, la partie métaphysaire est préférentiellement raccordée, par son bord interne, au col par une base de raccordement entre le col et la partie métaphysaire, ladite base présentant une surface d'appui limitant l'engagement de la tige dans un canal médullaire d'un fémur que l'on souhaite implanter.

Selon une autre caractéristique avantageuse de l'implant de l'invention, la partie métaphysaire comporte deux faces planes présentant des rainures longitudinales parallèles.

Selon une caractéristique additionnelle, l'implant fémoral de reprise proposé est tel que la partie métaphysaire comporte en retrait de son bord supéro-externe, des trous traversants s'ouvrant dans les deux faces planes et réservés au passage de liens destinés à cercler un volet osseux découpé dans la partie externe métaphysaire d'un fémur dans lequel l'implant est installé.

De préférence, la tige de l'implant présente une longueur axiale selon son axe principal (A-A') comprise entre 200 et 295 mm et l'axe principal de la tige (A-A') fait avec l'axe secondaire (B-B') du col un angle (α) compris entre 40° et 50°.

Enfin, la queue de l'implant présente de préférence, à distance de la partie métaphysaire au moins deux orifices, traversants, pratiqués dans le plan médian de l'implant et destinés au passage de clavettes de fixation.

L'implant de l'invention sera mieux compris à la lecture de la description faite ci-dessous en référence aux dessins annexés qui montrent, à titre d'exemples non limitatifs, des formes de réalisation de l'objet de l'invention et de sa mise en oeuvre.
- La **fig. 1** est une perspective de l'implant fémoral de reprise selon l'invention;
- La **fig. 2** est une vue de côté de l'implant de reprise de la **fig. 1****;**
- La **fig. 3** est une vue en coupe transversale prise selon la ligne III-III de la **fig. 2****;**
- La **fig. 4** est une vue de dessus de l'implant de reprise de la **fig. 2****;**
- La **fig. 5** est une vue de dos de l'implant de reprise des **fig. 1** **et** **2****;**
- La **fig. 6** représente selon un plan médio-latéral une coupe montrant l'implantation de l'implant de reprise dans un fémur et sa fixation ;
- La **fig. 7** représente en perspective un fémur implanté de l'implant de reprise de l'invention associé à un ancillaire d'implantation,
- la **fig. 8** est un agrandissement d'une partie de l'ancillaire d'implantation représenté sur la **fig. 7****.**

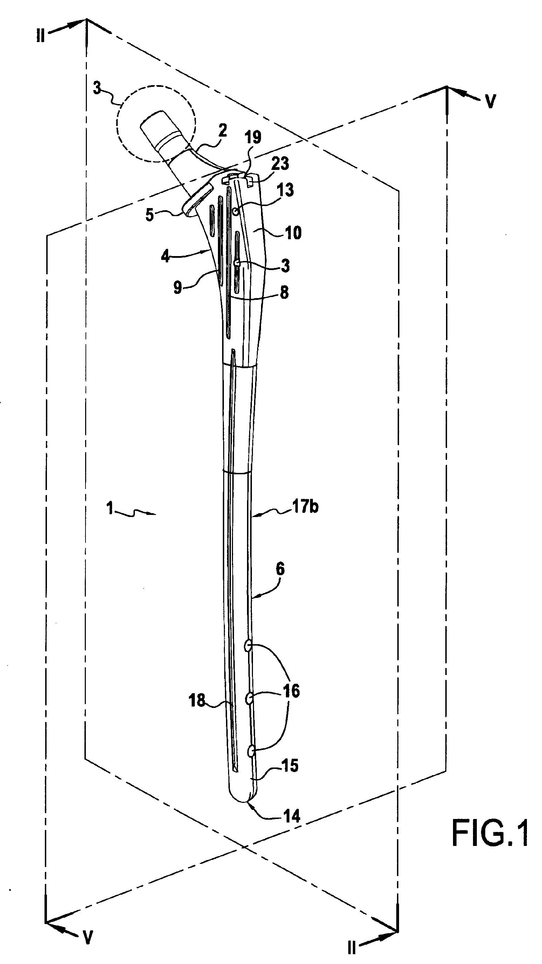

Selon les **fig. 1 à 5****,** l'implant fémoral de reprise de l'invention comprend essentiellement une tige **1,** souvent également appelée tige de révision, comprenant un col **2** formant une tête prothétique **3** d'articulation sur l'os du bassin et une queue **6** d'insertion dans le canal médullaire de l'os du fémur, le col **2** et la queue **6** étant liés par une partie massive **4,** dite métaphysaire, sensiblement triangulaire et s'étendant depuis une base **5** sensiblement plane de raccordement avec le col **2** pour se prolonger en son extrémité inférieure par la queue **6.**

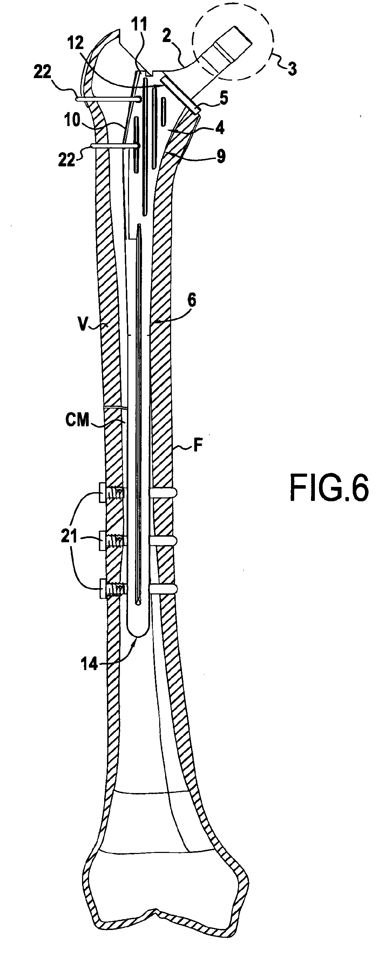

Comme représenté notamment sur les **fig.1** et **6****,** la tête prothétique **3** forme une sphère d'articulation de type rotule sur l'os du bassin. Une telle sphère peut de façon connue être formée de matière à l'extrémité du col **2** ou bien encore être constituée d'une sphère amovible comme illustré ici. Aussi dans le cadre de l'invention, la notion de tête **3** prothétique s'applique de façon indifférente à une tête intégrale sur le col **2** ou une tête amovible rapportée.

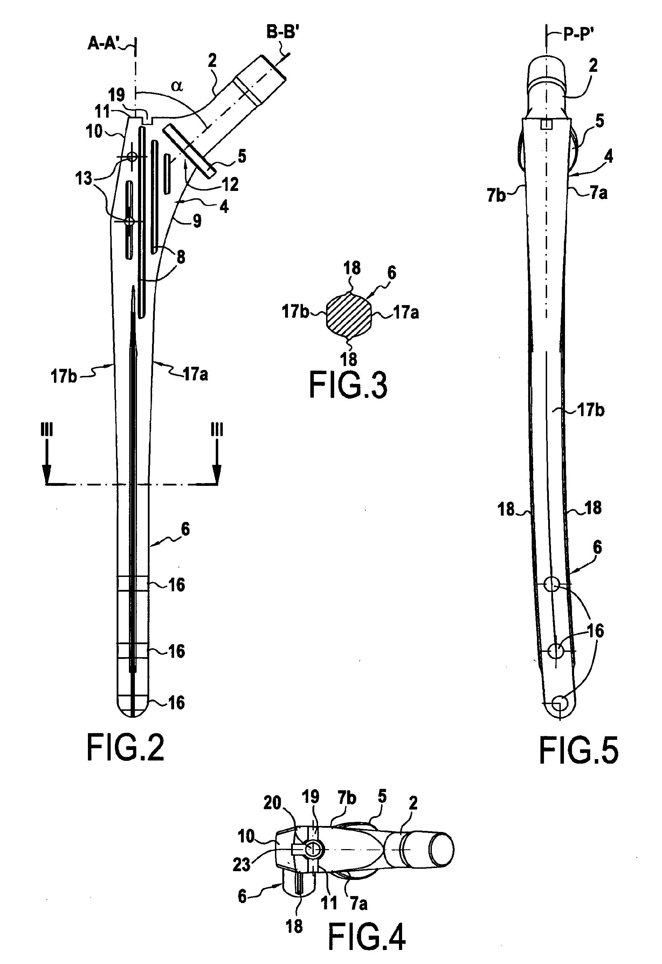

Comme cela ressort des **fig. 4** et **5****,** la partie métaphysaire **4** comporte deux faces planes **7a** et **7b** disposées de part et d'autre d'une surface médiane de l'implant **P-P',** dont la référence géométrique est à considérer en relation avec un plan frontal anatomique **II-II** sur la **Fig. 1****.** Bien que la tige **1** puisse être conformée pour présenter une courbure appropriée au fémur dans lequel elle doit être implantée, il est considéré, au sens de l'invention, que la surface référentielle **P-P'** est du type plan.

Les faces planes **7a** et **7b** sont, à partir de la base **5,** légèrement convergentes en direction du plan de référence frontal et présentent, chacune, des stries, rainures ou analogues **8,** établies longitudinalement et parallèlement entre elles, de façon à favoriser l'ancrage ultérieur de la masse osseuse de reconstitution après mise en place de l'implant de reprise dans le canal médullaire d'un fémur.

Pour favoriser encore l'ancrage après implantation, la partie métaphysaire **4** est de préférence avantageusement doublement revêtue de revêtement de titane poreux et d'hydroxyapatite. Les stries **8** sont du type non débouchant en extrémité proximale, afin d'éviter toute migration de particules vers le haut. En revanche, aux extrémités distales, les stries **8** sont "fuyantes", c'est-à-dire qu'elles tendent à se raccorder à la surface des faces **7a, 7b,** de manière à faciliter une rétraction future en cas de besoin.

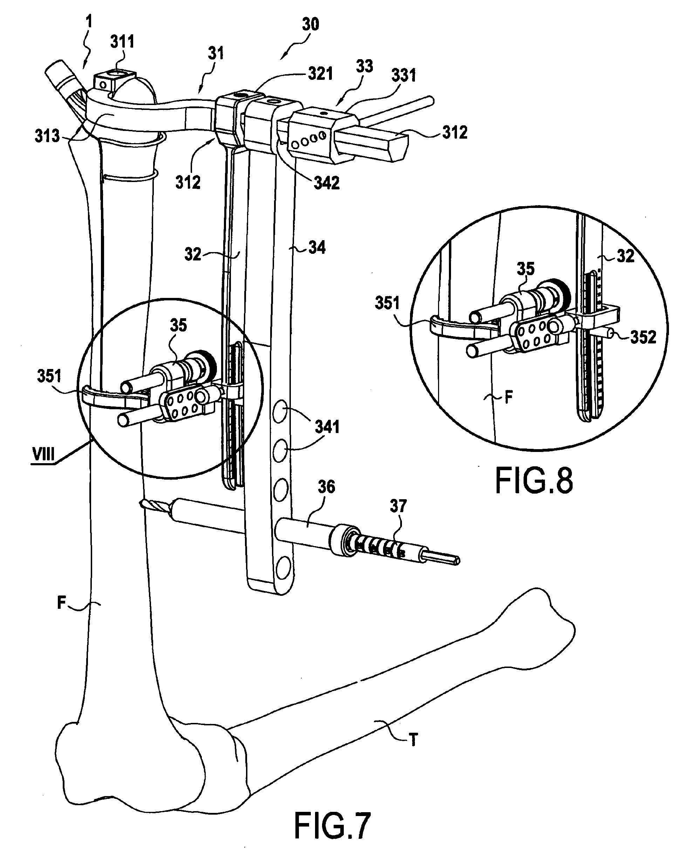

La partie métaphysaire **4** est délimitée en sa partie antérieure de raccordement du col **2** à la queue **6** par un bord interne **9,** de forme concave, s'étendant dans le prolongement du col **2** transversalement aux faces **7a, 7b** pour se raccorder à la queue **6.** De même, la partie métaphysaire **4** est délimitée en sa partie supéro-externe par un bord **10** plan et transversal aux faces **7a, 7b,** ainsi que par une surface plane **11** de raccordement à la base du col **2.** Dans la surface supérieure plane **11** est usinée une rainure débouchante **19** s'étendant entre les deux faces planes **7a, 7b** et au milieu de laquelle est percé un alésage borgne **20,** de préférence taraudé. Cette rainure **19** et cet alésage **20** sont destinés à permettre la fixation par encastrement d'un ancillaire **30** d'implantation comme il sera par la suite exposé en référence à la **fig. 7****.**

Comme indiqué sur la **fig. 2****,** la tige **1** s'étend globalement selon un axe longitudinal principal **A-A'** et le col **2** s'étend lui suivant un axe secondaire **B-B'** formant avec l'axe principal **A-A'** un angle a compris entre 40° et 50°, la base **5** dudit col étant quant à elle orientée sensiblement perpendiculairement à l'axe **B-B'** et formant de préférence, comme représenté sur les figures, une surface d'appui **12** dont la fonction sera expliquée ultérieurement.

La partie métaphysaire **4** présente, par ailleurs, en retrait de son bord externe **10,** des trous traversants **13** s'ouvrant sur les deux faces **7a** et **7b** et pratiqués, de préférence, de façon rectiligne et parallèlement l'un à l'autre, selon deux directions qui peuvent être appréciées comme étant perpendiculaires au plan **P-P'.**

Selon l'invention, la queue **6** de l'implant de reprise est conformée de façon appropriée pour augmenter la stabilité de l'implant lors de sa mise en place dans le canal médullaire du fémur.

La queue **6** présente une forme générale longiligne non cylindrique et non-droite et comporte deux faces planes **17a, 17b** parallèles entre elles et au plan sagittal **V-V** sur la **fig. 1****,** et reliés l'une à l'autre par deux faces non planes, en l'espèce des sections courbes convexes munies d'éléments longitudinaux anti-rotation tels qu'au moins un bourrelet ou ailette longitudinal **18** saillant sur la surface de la queue **6.**

De façon préférée, le nombre total d'éléments longitudinaux anti-rotation **18** est compris entre deux et six et ces éléments s'étendent depuis le niveau inférieur des rainures ou stries **8** sur la partie métaphysaire jusqu'à sensiblement l'extrémité distale **14** de la queue **6,** sans toutefois aller intégralement jusqu'à cette extrémité. Cette extrémité **14,** de forme générale arrondie, correspond, de préférence, à une partie terminale **15** globalement en forme d'ogive.

La longueur totale de la tige **1** de l'implant est de préférence comprise entre 200 et 295 mm, appréciée en étant prise entre la surface supérieure plane **11** de la partie métaphysaire **4** et l'extrémité **14** de la queue **6.**

En retrait de la partie terminale **15,** la queue **6** présente deux à trois trous **16** traversants, parallèles entre eux et qui sont pratiqués selon des directions perpendiculaires aux plats **17a, 17b** et donc perpendiculaires au plan sagittal **V-V.**

A titre d'exemple, l'implant de reprise selon l'invention peut être réalisée en alliage de titane TA6V selon ISO 5832-3 ou en acier implantable du type acier inoxydable Z6 CNM 21-09 forgeable (M30NW) selon ISO 5832-9.

L'implant de reprise décrit ci-dessus est destiné à être implanté dans un fémur **F** dont le canal médullaire **CM** a fait l'objet d'une remise en état naturel ou d'un nettoyage destiné à éliminer les traces de liant ou de ciment correspondant à une implantation antérieure. En particulier, l'implant de reprise de l'invention peut être destiné aux opérations de révision avec volet fémoral **V** comme décrit ci-après en référence à la **fig. 6****.**

Le canal médullaire **CM** est traité sur une longueur supérieure à celle de l'implant à reprendre. Afin d'accéder à la partie diaphysaire du canal médullaire **CM,** on taille dans la partie métaphysaire externe du fémur, un volet **V** offrant un accès à la partie interne métaphysaire et surtout un accès en profondeur au canal médullaire **CM.**

Lorsque les opérations préalables de remise en état dudit canal médullaire **CM** ont été assurées, il est aussi prévu de conférer au canal médullaire **CM** une section droite transversale supérieure à celle de la queue **6,** voire d'adapter, par résection, la face interne de la corticale de la partie métaphysaire du fémur, pour autoriser un appui réparti de coopération avec le bord interne concave **9.** Il peut aussi être envisagé de retailler le plan de résection initial, de manière à lui conférer un état favorisant un appui éventuel avec la surface d'appui **12** de la base **5** du col **2** qui constitue alors une butée limitant l'engagement de la tige dans le canal médullaire et permettant de transmettre des contraintes à l'os afin que ce dernier se redynamise.

L'implant selon l'invention est implanté sans ciment mais bloqué en position par un verrouillage distal consistant à exécuter, dans la partie distale externe du fémur **F,** des trous de passage de deux ou trois clavettes **21** qui sont engagées, par ces trous, à l'intérieur des trous traversants **16** de la queue **6** de l'implant pour être vissées directement dans la corticale du fémur. De plus, pour favoriser la reconstruction osseuse et la stabilité de l'implant, celui-ci est entièrement revêtu de préférence d'hydroxyapatite avant implantation.

Comme cela ressort de la **fig. 6****,** l'implant selon l'invention est alors implanté directement dans le canal médullaire **CM,** en étant verrouillé en partie distale avec appui simultané du bord interne concave **9** sur la face interne de la corticale interne de la partie métaphysaire, appui éventuellement complété par l'arrêt ou la butée assuré par la patte d'appui **12.**

Le volet **V** préalablement découpé est alors remis en place et lié, pour une consolidation osseuse ultérieure, par deux liens engagés dans les trous traversants **13,** de façon à établir, par passage au travers du trait de coupe ayant permis le détachement du volet **V,** deux ceintures ou cerclages **22** solidarisant le volet **V** à la surface supéro-externe de la partie métaphysaire **4** dans une position stable qui est conférée par la coopération entre le bord interne concave **9** et la corticale interne du fémur et par le verrouillage établi par les vis **21.** Dans cette position stable, la formation osseuse rétablit rapidement l'intégrité entre le fémur et le volet **V.**

Les dispositions structurelles rappelées, attachées à l'implant de reprise ci-dessus, permettent de réaliser la réhabilitation d'une implantation prothétique antérieure connaissant des altérations ou des désordres, sans qu'une telle réimplantation ait à souffrir des désordres de l'implantation précédente. En effet, par la découpe du volet **V,** il est possible d'atteindre un matériel osseux de bonne qualité dans la partie distale profonde du fémur **F** et de réaliser un verrouillage maintenant, en raison de la grande longueur de la queue **6,** une liaison ferme et résistante favorisant l'appui du bord **9** sur la face interne de la corticale interne de la partie métaphysaire du fémur.

De cette manière, une reconstitution du matériel osseux interne au canal médullaire peut intervenir dans les meilleures conditions, de même que le rétablissement de l'intégrité de la partie métaphysaire du fémur par reconsolidation du volet **V** immobilisé en position stable sur la partie métaphysaire de l'implant par l'intermédiaire des cerclages **22.**

Il doit, bien entendu, être considéré que les clavettes **21** peuvent être prévues pour établir un verrouillage temporaire ou permanent.

Afin d'assurer une implantation précise et stable de la prothèse de reprise, un ancillaire **30** d'implantation dédié spécifiquement aux opérations chirurgicales de positionnement et ancrage de l'implant de reprise dans le canal médullaire du fémur est nécessaire. Un tel ancillaire **30** est représenté sur la **fig. 7****.** Il comporte essentiellement une potence **31** comportant à une extrémité un organe **311** de fixation par encastrement sur la surface supérieure plane **11** de la partie métaphysaire **4** de l'implant, cet organe **311** étant configuré pour s'encastrer en correspondance des rainures **19** et **23** et du trou **20** qui y sont formés. Ces moyens complémentaires de fixation par encastrement représentent des références d'adaptation et fixation temporaires de l'ancillaire **30** sur l'implant de reprise lors de son positionnement dans le canal médullaire **CM** du fémur **F.**

La potence **31** de l'ancillaire **30** comporte également un fût **312** rectiligne de montage et réglage d'au moins un outil de positionnement de l'implant de reprise dans le canal médullaire d'un fémur. De façon originale, le fût et l'organe de fixation de la potence sont reliés par une partie coudée **313** permettant un contournement antérieur ou postérieur du grand trochanter **T** lorsque la potence **31** est encastrée sur la surface plane **11** de l'implant inséré dans le canal médullaire du fémur **F.**

La potence **31** de l'ancillaire **30** sert de référence pour le placement d'outils de positionnement de l'implant décrits ci-après. L'ancillaire **30** comporte notamment au moins un guide d'enfoncement **32** muni d'une réglette et un guide d'antéversion **33,** tous deux aptes à être montés sur le fût de la potence par une tête de montage **321, 331** femelle de section interne complémentaire de la section externe du fût **312** et telle que le guide d'enfoncement **32** et le guide d'antéversion **33** s'étendent perpendiculairement à l'axe du fût. Le guide d'antéversion **33** sert à positionner en rotation la tige **1** en fonction de critères d'anatomie recherchés par le chirurgien. Sa spécificité est qu'en révision les repères anatomiques du fémur peuvent être altérés et que son positionnement permet de se positionner en utilisant d'autres repères, tel le tibia **T.** Le guide d'enfoncement **32** a pour utilité, qu'une fois l'enfoncement et l'antéversion choisis avec un implant d'essai, la position de l'implant définitif soit retrouvée, en se basant à la position relative entre le guide d'enfoncement **32** et un davier fémoral **35.**

L'ancillaire **30** comporte par ailleurs de façon avantageuse un tel davier **35** de repérage équipé de mors **351** de fixation sur le fémur implanté et une platine de fixation d'une tige **352** repère d'enfoncement.

Monté sur la potence **31,** un guide de perçage **34** distal comportant des orifices de perçage **341** est également prévu pour le perçage en aveugle de la partie diaphysaire du fémur **F** en exacte correspondance avec les trous **16** lorsque l'implant est mis en place dans le canal médullaire **CM.** Le guide de perçage **34,** comme les guides d'enfoncement et d'antéversion, est apte à être monté sur la potence par une tête de montage femelle **342** de section interne complémentaire de la section externe du fût **312** de la potence **31** et telle que le guide de perçage **34** s'étende perpendiculairement au fût et parallèlement à l'axe principal **A-A'** de l'implant de reprise enfoncé dans le canal médullaire **CM** du fémur **F.**

L'ancillaire **30** comporte enfin des canons **36** et de mèches **37** de perçage aptes à être insérés dans les orifices de perçage **341** du guide de perçage **34** et qui permettent, une fois les dispositions de réglages adéquates prises à l'aide des autres guides **32,33** et davier **35,** de percer le fémur aux positions exactes voulue pour verrouiller l'implant à l'aide des clavettes **21** de verrouillage.

## Revendications

1. Implant fémoral de reprise, comprenant une tige (**1**) liée par un col (**2**) à une tête (**3**), ladite tige comportant, à partir du col, une partie métaphysaire (**4**), prolongée par une queue (**6**), la tige possédant un axe principal (**A-A'**) faisant un angle (a) avec un axe secondaire **(B-B')** du col et la queue (**6**) présentant deux faces planes (**17a**, **17b**) parallèles entre elles et à un plan sagittal (**V-V**) de l'implant, ledit implant étant **caractérisé en ce que** lesdites faces planes sont reliés l'une à l'autre par deux faces non planes munies d'éléments longitudinaux anti-rotation (**18**).

2. Implant fémoral de reprise selon la revendication 1, **caractérisé en ce que** les éléments longitudinaux anti-rotation (**18**) sont constitués d'ailettes ou de bourrelets saillants à la surface des faces non-planes de la queue (**6**) de la tige (**1**).

3. Implant fémoral de reprise selon la revendication 1 ou la revendication 2, **caractérisé en ce qu'**il comporte au moins deux éléments longitudinaux anti-rotation (**18**), et de préférence entre deux et six éléments longitudinaux anti-rotation (**18**).

4. Implant fémoral de reprise selon la revendication 1 ou la revendication 2, **caractérisé en ce que** les éléments longitudinaux anti-rotation (**18**) s'étendent depuis la base de la queue (**6**) jusqu'à sensiblement l'extrémité distale (**14**) de la queue.

5. Implant fémoral de reprise selon l'une des revendications 1 à 4 **caractérisé en ce que** la partie métaphysaire (**4**) comporte :
- deux faces planes (**7a**, **7b**),
- un bord interne (**9**) concave, raccordant le col à la queue,
- et un bord supéro-externe(**10**) prenant naissance à partir d'une surface supérieure plane (**11**) de raccordement au col (**2**).

6. Implant fémoral de reprise selon la revendication 5, **caractérisé en ce que** la partie métaphysaire (**4**) est raccordée, par son bord interne (**9**), au col par une base (**5**) de raccordement entre le col et la partie métaphysaire, ladite base (**5**) présentant une surface d'appui (**12**) limitant l'engagement de la tige (**1**) dans un canal médullaire.

7. Implant fémoral de reprise selon l'une des revendications 1 à 6, **caractérisé en ce que** la partie métaphysaire (**4**) comporte deux faces planes (**7a**, **7b**) présentant des rainures longitudinales parallèles (**8**).

8. Implant fémoral de reprise selon l'une des revendications 5 à 7, **caractérisé en ce que** la partie métaphysaire (**4**) comporte en retrait de son bord supéro-externe (**10**), des trous traversants (**13**) s'ouvrant dans les deux faces planes (**7a**, **7b**) et réservés au passage de liens (**22**) destinés à cercler un volet osseux (**V**) découpé dans la partie externe métaphysaire d'un fémur (**F**) dans lequel l'implant est installé.

9. Implant fémoral de reprise selon l'une des revendications 1 à 8, **caractérisé en ce que** la tige (**1**) présente une longueur axiale selon son axe principal (**A-A**') comprise entre 200 et 295 mm.

10. Implant fémoral de reprise selon l'une des revendications 1 à 9, **caractérisé en ce que** l'axe principal de la tige (**A-A'**) fait avec l'axe secondaire (**B-B'**) du col (**2**) un angle (a) compris entre 40° et 50°.

11. Implant fémoral de reprise selon l'une des revendications 1 à 10, **caractérisé en ce que** la queue présentent, à distance de la partie métaphysaire au moins deux orifices (**16**), traversants, pratiqués dans le plan médian de l'implant et destinés au passage de clavettes de fixation (**21**).

## Patentansprüche

1. Femurimplantat zur Ausbesserung, umfassend eine Stange (1), die durch einen Hals (2) mit einem Kopf (3) verbunden ist, wobei die Stange von dem Hals ausgehend einen metaphysären Teil (4), welcher durch einen Schaft (6) fortgesetzt ist, umfasst, wobei die Stange eine Hauptachse (A-A') besitzt, die mit einer Nebenachse (B-B') des Halses einen Winkel (α) einschließt, und wobei der Schaft (6) zwei plane Seiten (17a, 17b) aufweist, die zueinander und zu einer Sagittalebene (V-V) des Implantats parallel verlaufen, wobei das Implantat **dadurch gekennzeichnet ist, dass** die planen Seiten durch zwei nicht plane Seiten, welche mit längsverlaufenden Drehsicherungselementen (18) ausgestattet sind, miteinander verbunden sind.

2. Femurimplantat zur Ausbesserung nach Anspruch 1, **dadurch gekennzeichnet, dass** die längsverlaufenden Drehsicherungselemente (18) von Flügeln oder von Wülsten gebildet sind, die an der Oberfläche der nicht planen Seiten des Schafts (6) der Stange (1) vorspringen.

3. Femurimplantat zur Ausbesserung nach Anspruch 1 oder Anspruch 2, **dadurch gekennzeichnet, dass** es wenigstens zwei längsverlaufende Drehsicherungselemente (18) und vorzugsweise zwischen zwei und sechs längsverlaufende Drehsicherungselemente (18) umfasst.

4. Femurimplantat zur Ausbesserung nach Anspruch 1 oder Anspruch 2, **dadurch gekennzeichnet, dass** die längsverlaufenden Drehsicherungselemente (18) sich von der Basis des Schafts (6) bis im Wesentlichen zu dem distalen Ende (14) des Schafts erstrecken.

5. Femurimplantat zur Ausbesserung nach einem der Ansprüche 1 bis 4, **dadurch gekennzeichnet, dass** der metaphysäre Teil (4) umfasst:
- zwei plane Seiten (7a, 7b),
- einen konkaven Innenrand (9), der den Hals mit dem Schaft verbindet, und
- einen superolateralen Rand (10), der aus einer planen oberen Fläche (11) zum Anschließen an den Hals (2) entspringt.

6. Femurimplantat zur Ausbesserung nach Anspruch 5, **dadurch gekennzeichnet, dass** der metaphysäre Teil (4) mit seinem Innenrand (9) durch einen Sockel (5) zur Verbindung zwischen dem Hals und dem metaphysären Teil an den Hals angeschlossen ist, wobei der Sockel (5) eine Anlagefläche (12) aufweist, die das Eingreifen der Stange (1) in einen Markkanal begrenzt.

7. Femurimplantat zur Ausbesserung nach einem der Ansprüche 1 bis 6, **dadurch gekennzeichnet, dass** der metaphysäre Teil (4) zwei plane Seiten (7a, 7b), die parallele Längsnuten (8) aufweisen, umfasst.

8. Femurimplantat zur Ausbesserung nach einem der Ansprüche 5 bis 7, **dadurch gekennzeichnet, dass** der metaphysäre Teil (4) von seinem superolateralen Rand (10) zurückliegend Durchgangslöcher (13) aufweist, die sich in den beiden planen Seiten (7a, 7b) öffnen und die für den Durchgang von Verbindungsteilen (22) vorgesehen sind, welche dazu bestimmt sind, einen Knochendeckel (V) zu umfassen, der aus dem metaphysären Außenteil eines Oberschenkelknochens (F), in den das Implantat eingesetzt ist, ausgeschnitten ist.

9. Femurimplantat zur Ausbesserung nach einem der Ansprüche 1 bis 8, **dadurch gekennzeichnet, dass** die Stange (1) eine axiale Länge entlang ihrer Hauptachse (A-A') im Bereich zwischen 200 und 295 mm aufweist.

10. Femurimplantat zur Ausbesserung nach einem der Ansprüche 1 bis 9, **dadurch gekennzeichnet, dass** die Hauptachse der Stange (A-A') mit der Nebenachse (B-B') des Halses (2) einen Winkel (α) zwischen 40° und 50° einschließt.

11. Femurimplantat zur Ausbesserung nach einem der Ansprüche 1 bis 10, **dadurch gekennzeichnet, dass** der Schaft im Abstand von dem metaphysären Teil wenigstens zwei Durchgangsöffnungen (16) aufweist, die in der Medianebene des Implantats ausgebildet und für den Durchgang von Befestigungskeilen (21) bestimmt sind.

## Claims

1. A femur repair implant comprising a shank (1) connected by a neck (2) to a head (3), said shank comprising, starting from the neck, a metaphyseal portion (4) extended by a stem (6), the shank possessing a main axis (A-A') at an angle (α) relative to a secondary axis (B-B') of the neck, and the stem (6) presenting two plane faces (17a, 17b) that are parallel to each other and to a sagittal plane (V-V) of the implant, said implant being **characterized in that** said plane faces are connected together by two non-plane faces provided with longitudinal anti-rotation elements (18).

2. A femur repair implant according to claim 1, **characterized in that** the longitudinal anti-rotation elements (18) are constituted by fins or beads protecting from the surfaces of the non-plane faces of the stem (6) of the shank (1).

3. A femur repair implant according to claim 1 or claim 2, **characterized in that** it includes at least two longitudinal anti-rotation elements (18) and preferably two to six longitudinal anti-rotation elements (18).

4. A femur repair implant according to claim 1 or claim 2, **characterized in that** the longitudinal anti-rotation elements (18) extend from the base of the stem (6) substantially as far as the distal end (14) of the stem.

5. A femur repair implant according to any one of claims 1 to 4, **characterized in that** the metaphyseal portion (4) comprises:
· two plane faces (7a, 7b);
· a concave inner edge (9) connecting the neck to the stem; and
· a top-external edge (10) starting from a plane top surface (11) for junction to the neck (2).

6. A femur repair implant according to claim 5, **characterized in that** the metaphyseal portion (4) is joined by its inner edge (9) to the neck via a junction base (5) between the neck and the metaphyseal portion, said base (5) presenting a bearing surface (12) limiting engagement of the shank (1) in a medullary canal.

7. A femur repair implant according to any one of claims 1 to 6, **characterized in that** the metaphyseal potion (4) has two plane faces (7a, 7b) presenting parallel longitudinal groves (8).

8. A femur repair implant according to any one of claims 5 to 7, **characterized in that** the metaphyseal portion (4) includes through holes (13) set back from its top-external edge (10), opening out into the two plane faces (7a, 7b), and reserved for passing ties (22) for encircling a section of bone (V) cut out from the external metaphyseal portion of a femur (F) in which the implant is installed.

9. A femur repair implant according to any one of claims 1 to 8, **characterized in that** the shank (1) presents an axial length along its main axis (A-A') lying in the range 200 mm to 295 mm.

10. A femur repair implant according to any one of claims 1 to 9, **characterized in that** the main axis (A-A') of the shank lies at an angle (α) relative to the secondary axis (B-B') of the neck (2), which angle (α) lies in the range 40° to 50°.

11. A femur repair implant according to any one of claims 1 to 10, **characterized in that**, at a distance from the metaphyseal portion, the stem presents at least two through orifices (16) formed in the midplane of the implant for the purpose of passing fastener pins (21).
